# EUROPEAN PATENT APPLICATION

(11) **EP 3 345 530 A1**
(43) Date of publication of application: **11.07.2018**
(21) Application number: 16841806.9
(22) Date of filing: 29.08.2016
(51) Int. Cl.: A61B 1/04, A61B 1/00

(54) **IMAGING SYSTEM, PROCESSING DEVICE, PROCESSING METHOD, AND PROCESSING PROGRAM**

(30) Priority: 01.09.2015 JP 2015171927
(71) Applicant: Olympus Corporation, Hachioji-shi Tokyo 192-8507 (JP)
(72) Inventor: ITO, Takehiko, Hachioji-shi Tokyo 192-8507 (JP)
(74) Representative: Gunzelmann, Rainer
(86) International application number: PCT/JP2016/075218
(87) International publication number: WO 2017/038774

(57) **Abstract**

An endoscopic system 1 according to the present invention is provided with: an endoscope 2 which includes a left-eye optical system 21A, a right-eye optical system 21B, an imaging unit 20, a memory 24 storing view angle correction data; and a light source integrated-type processor 3 which includes a correction data acquisition unit 311 that acquires the view angle correction data from the memory 24 and a correction unit 35 that performs corrections based on the acquired view angle correction data such that a view angle of a left-eye image and a view angle of a right-eye image match each other and a size of the left-eye image and a size of the right-eye image match each other. Accordingly, provided is an imaging system capable of suitably executing image corrections in response to a mounted optical unit even if an arbitrary optical unit is mounted to a processing device as the optical unit including two optical systems for stereoscopic observation.

## Description

### Field

The present invention relates to an imaging system, a processing device, a processing method, and a processing program.

### Background

Recently, an integrated stereoscopic camera, which is provided with two image sensors corresponding to both eyes and two optical systems corresponding to the respective image sensors and is configured to capture a subject and obtain a stereoscopic image using two right and left image data thus captured, has been proposed as a digital camera (for example, see Patent Literature 1). Such a stereoscopic camera holds a correction parameter corresponding to individual differences of the two mounted optical systems, and has a function of correcting mismatch in size between a left-eye image data and a right-eye image data caused by the individual differences of the two optical systems inside the stereoscopic camera using this correction parameter.

Meanwhile, there is a demand for observation of an observation target in a stereoscopic image, which is provided with an endoscopic system to and from which an endoscope and a processing device (processor) in a detachable manner and used in medical fields and the like, in order for facilitation of diagnosis and inspection. As a technique to meet the demand, an endoscope, which is provided with an optical unit including two optical systems for stereoscopic observation and two image sensors corresponding to the respective optical units, has been known. The processor to which the endoscope is mounted receives right and left image data from the mounted endoscope and generates a stereoscopic image using the two image data.

### Citation List

### Patent Literature

Patent Literature 1: JP 2006-162991 A

### Summary

### Technical Problem

Endoscopes have individual differences, and a variation in size between images caused by the individual differences of the two optical systems of the endoscope varies depending on the mounted endoscope. Thus, it is difficult for the processor to perform image correction suitable for the mounted endoscope.

The present invention has been made in view of the above-described problem, and an object thereof is to provide an imaging system, a processing device, a processing method, and a processing program which is capable of suitably executing image correction in response to a mounted optical unit even if an arbitrary optical unit is mounted to a processing device as the optical unit including two optical systems for stereoscopic observation. Solution to Problem

To solve the problem described above and to achieve the object, an imaging system according to the present invention includes: an optical unit; a processing device having a configuration to which the optical unit is mounted in a detachable manner; and an imaging device which is arranged in either the optical unit or the processing device. The optical unit includes: a first optical system configured to image light incident from a subject; a second optical system configured to image the light incident from the subject with a parallax from the first optical system; and a first storage unit configured to store view angle correction data for correction of a difference between a view angle of the first optical system and a view angle of the second optical system. The imaging device includes: a first imaging unit configured to capture an optical image imaged by the first optical system to generate first image data; and a second imaging unit configured to capture an optical image imaged by the second optical system to generate second image data. The processing device includes: a data acquisition unit configured to acquire the view angle correction data from the first storage unit; and a correction unit configured to correct at least one of the first image data and the second image data based on the view angle correction data acquired by the data acquisition unit such that a view angle of a first image corresponding to the first image data and a view angle of a second image corresponding to the second image data match each other, and a size of the first image and a size of the second image match each other.

In the imaging system according to the present invention, the view angle correction data includes at least instruction data, which instructs the correction unit to cause the view angle and the size of the second image to match the view angle and the size of the first image, and a first magnification of the view angle of the second image relative to the view angle of the first image.

In the imaging system according to the present invention, the processing device further includes: a display unit configured to display and output an image processed by the processing device; and a parameter generation unit configured to generate a first scale factor to be used in the correction unit based on a second magnification, which is a scale factor set in advance according to a type of the optical unit and a standard of the display unit, and a second scale factor to be used in the correction unit based on the first magnification acquired by the data acquisition unit and the second magnification, and the correction unit includes: an area setting unit configured to set a size of a first extraction area to be extracted from the first image based on an extraction reference area from each image, and set a size of a second extraction area to be extracted from the second image based on the extraction reference area and the first magnification acquired by the data acquisition unit; and a scaling unit configured to extract the first extraction area from the first image and output, as a corrected first image, an image obtained by enlarging or reducing the extracted first extraction area using the first scale factor, and further, extract the second extraction area from the second image and output, as a corrected second image, an image obtained by enlarging or reducing the extracted second extraction area using the second scale factor.

In the imaging system according to the present invention, the processing device further includes a second storage unit configured to store the second magnification to be associated with each of the type of the optical unit and the standard of the display unit, the first storage unit is configured to store identification information indicating the type of the optical unit, the data acquisition unit is configured to acquire the identification information of the optical unit from the first storage unit, and acquire the second magnification according to the type of the optical unit and the standard of the display unit indicated by the acquired identification information, from the second storage unit, and the parameter generation unit is configured to generate the first scale factor and the second scale factor based on the first magnification and the second magnification acquired by the data acquisition unit.

In the imaging system according to the present invention, the view angle correction data includes first position data indicating a position of the extraction reference area in the first image and second position data indicating a position of the extraction reference area in the second image, and the area setting unit is configured to set the position of the extraction reference area in the first image based on the first position data acquired by the data acquisition unit and set the position of the extraction reference area in the second image based on the second position data acquired by the data acquisition unit.

In the imaging system according to the present invention, each of the first optical system and the second optical system has an optical zoom function to change the view angle, and the correction unit is configured to control the optical zoom function of at least any one of the first optical system and the second optical system based on the view angle correction data acquired by the data acquisition unit so as to cause the view angle of the first image corresponding to the first image data and the view angle of the second image corresponding to the second image data to match each other and the size of the first image and the size of the second image to match each other.

The imaging system according to the present invention includes: an endoscopic device including the optical unit and the imaging device; and the processing device configured to process image data captured by the imaging device.

A processing device according to the present invention is a processing device to which an optical unit is mounted in a detachable manner and configured to process image data output from an imaging device, the optical unit including a first optical system configured to image light incident from a subject, a second optical system configured to image the light incident from the subject with a parallax from the first optical system, and a first storage unit configured to store view angle correction data for correction of a difference between a view angle of the first optical system and a view angle of the second optical system, the imaging device being arranged in either the optical unit or the processing device and including a first imaging unit configured to capture an optical image imaged by the first optical system to generate first image data and a second imaging unit configured to capture an optical image imaged by the second optical system to generate second image data. The processing device includes: a data acquisition unit configured to acquire the view angle correction data from the first storage unit of the optical unit mounted to the processing device; and a correction unit configured to correct at least one of the first image data and the second image data based on the view angle correction data acquired by the data acquisition unit such that a view angle of a first image corresponding to the first image data and a view angle of a second image corresponding to the second image data match each other, and a size of the first image and a size of the second image match each other.

A processing method according to the present invention is a processing method that is executed by a processing device to which an optical unit including a first optical system that images light incident from a subject and a second optical system that images the light incident from the subject with a parallax from the first optical system is mounted in a detachable manner, the processing device being configured to process image data output from a first imaging unit configured to capture an optical image imaged by the first optical system to generate first image data and a second imaging unit configured to capture an optical image imaged by the second optical system to generate second image data. The processing method includes: a data acquisition process of acquiring view angle correction data for correction of a difference between a view angle of the first optical system and a view of the second optical system, from a first storage unit included in the optical unit mounted to the processing device; and a correction process of correcting at least one of the first image data and the second image data based on the view angle correction data acquired in the data acquisition process such that a view angle of a first image corresponding to the first image data and a view angle of a second image corresponding to the second image data match each other, and a size of the first image and a size of the second image match each other.

A processing program according to the present invention is a processing program that causes a processing device to which an optical unit including a first optical system that images light incident from a subject and a second optical system that images the light incident from the subject with a parallax from the first optical system is mounted in a detachable manner, the processing device being configured to process image data output from a first imaging unit configured to capture an optical image imaged by the first optical system to generate first image data and a second imaging unit configured to capture an optical image imaged by the second optical system to generate second image data, to execute: a data acquisition procedure of acquiring view angle correction data for correction of a difference between a view angle of the first optical system and a view angle of the second optical system, from a first storage unit included in the optical unit mounted to the processing device; and a correction procedure of correcting at least one of the first image data and the second image data based on the view angle correction data acquired in the data acquisition procedure such that a view angle of a first image corresponding to the first image data and a view angle of a second image corresponding to the second image data match each other, and a size of the first image and a size of the second image match each other. Advantageous Effects of Invention

According to the invention, it is possible to suitably execute image correction in response to the mounted optical unit even if an arbitrary optical unit is mounted to the processing device as the optical unit including the two optical systems for stereoscopic observation.

### Brief Description of Drawings

FIG. 1 is a schematic view illustrating a schematic configuration of an endoscopic system according to an embodiment of the invention.
FIG. 2 is a flowchart illustrating a processing procedure of a process executed with respect to image data input from an endoscope by a light source integrated-type processor illustrated in FIG. 1.
FIG. 3 is a flowchart illustrating a process procedure of a correction process illustrated in FIG. 2.
FIG. 4 is a view for describing a reference area position setting process illustrated in FIG. 3.
FIG. 5 is a view for describing an extraction area size setting process illustrated in FIG. 3.
FIG. 6 is a view for describing a scaling process illustrated in FIG. 3.
FIG. 7 is a view for describing the reference area position setting process illustrated in FIG. 3.
FIG. 8 is a view for describing the extraction area size setting process illustrated in FIG. 3.
FIG. 9 is a view for describing the scaling process illustrated in FIG. 3.
FIG. 10 is a schematic view illustrating another schematic configuration of an imaging system according to an embodiment of the invention.

### Description of Embodiments

In the following description, a medical endoscopic system will be described as a mode for carrying out the present invention (hereinafter, referred to as an "embodiment"). In addition, the invention is not limited by the embodiments. Further, the same parts are denoted by the same reference signs when the drawings are described.

### (Embodiment)

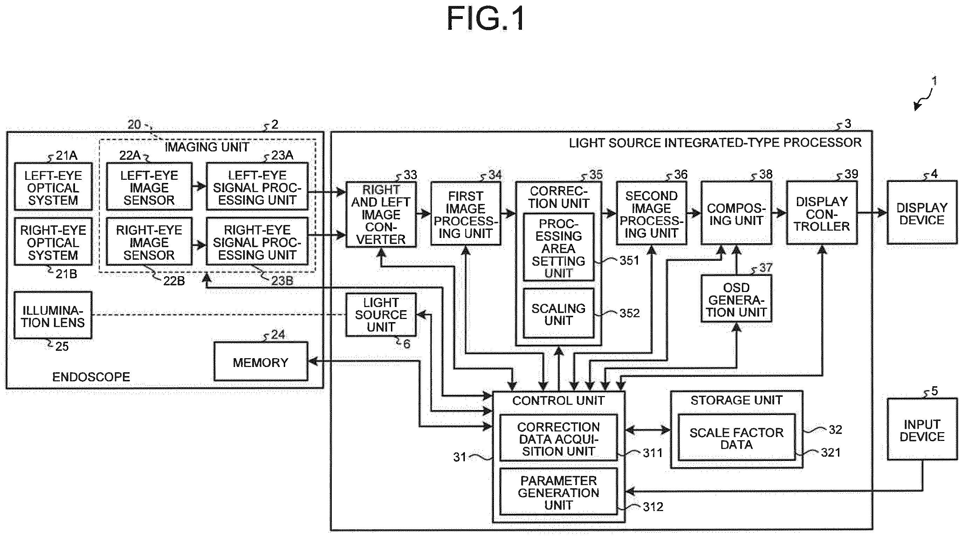

FIG. 1 is a schematic view illustrating a schematic configuration of an endoscopic system according to an embodiment of the invention. As illustrated in FIG. 1, an endoscopic system 1 according to the present embodiment includes: an endoscope 2 for introduction into a subject; a light source integrated-type processor 3 (processing device) which performs predetermined image processing with respect to an capturing signal transmitted from the mounted endoscope 2 (imaging device) via a connector (not illustrated); a display device 4 (display unit) which displays a stereoscopic image corresponding to the capturing signal from the endoscope 2; and an input device 5 which receives input of various types of instruction information and inputs the instruction information to the light source integrated-type processor 3. The light source integrated-type processor 3 has a configuration to which the endoscope 2 is mounted in a detachable manner via the connector. The light source integrated-type processor 3 is a processor which includes a light source unit 6 therein.

The endoscope 2 includes a flexible insertion portion to be inserted into the subject, and image data inside the subject obtained by capturing the inside of the subject's body is generated by an imaging unit 20 that is provided at a distal end portion of the insertion portion. The endoscope 2 includes a left-eye optical system 21A (first optical system), a right-eye optical system 21B (second optical system), a left-eye image sensor 22A (first imaging unit), the imaging unit 20, a memory 24 (first storage unit), and an illumination lens 25 at the distal end portion. The imaging unit 20 includes a left-eye image sensor 22A (first imaging unit), and a right-eye image sensor 22B (second imaging unit), a left-eye signal processing unit 23A, and a right-eye signal processing unit 23B. The endoscope 2 includes an illumination fiber (light guide cable) and an electrical cable (not illustrated) extending from the distal end to a connector (not illustrated) at a proximal end thereof. In addition, the endoscope 2 includes an operation switch unit (not illustrated) provided with various operation switches.

The left-eye optical system 21A includes one or a plurality of lenses, is provided on the front side of the left-eye image sensor 22A, and images light incident from the subject.

The right-eye optical system 21B includes one or a plurality of lenses, is provided on the front side of the right-eye image sensor 22B, and images the light incident from the subject with a parallax from the left-eye optical system 21A. Incidentally, each of the left-eye optical system 21A and the right-eye optical system 21B may have an optical zoom function of changing an angle of view and a focus function of changing focus.

The left-eye image sensor 22A captures an optical image imaged by the left-eye optical system 21A and generates left-eye image data (first image data). The left-eye image sensor 22A is a CMOS image sensor or a CCD image sensor, and a plurality of pixels, which receive light from the subject to which light has been incident, and perform photoelectric conversion of the received light to generate image data, are arranged in a matrix form on a light receiving surface thereof.

The right-eye image sensor 22B captures an optical image imaged by the right-eye optical system 21B and generates right-eye image data (second image data). The right-eye image sensor 22B is a CMOS image sensor or a CCD image sensor, and a plurality of pixels, which receive light from the subject to which light has been incident, and perform photoelectric conversion of the received light to generate image data, are arranged in a matrix form on a light receiving surface thereof.

The left-eye signal processing unit 23A includes an analog processing unit which performs noise removal processing and clamp processing and an A/D converter which performs A/D conversion processing, with respect to the left-eye image data (analog) output from the left-eye image sensor 22A, and outputs left-eye image data (digital) to the light source integrated-type processor 3. Incidentally, there is also a configuration in which the left-eye signal processing unit 23A is provided on the light source integrated-type processor 3 side.

The right-eye signal processing unit 23B includes an analog processing unit which performs noise removal processing and clamp processing and an A/D converter which performs A/D conversion processing, with respect to the right-eye image data (analog) output from the right-eye image sensor 22B, and outputs right-eye image data (digital) to the light source integrated-type processor 3. Incidentally, there is also a configuration in which the right-eye signal processing unit 23B is provided on the light source integrated-type processor 3 side.

The memory 24 records identification information which indicates a type and a model number of the endoscope 2, and types of the left-eye image sensor 22A and the right-eye image sensor 22B, and the like. The memory 24 stores view angle correction data for correction of a difference between a view angle of the left-eye optical system 21A and a view angle of the right-eye optical system 21B. The view angle correction data is data depending on each of the endoscopes 2. The view angle correction data includes instruction data which instructs a correction unit 35 to cause a view angle and a size of a right-eye image to match a view angle and a size of a left-eye image between the left-eye image (first image) corresponding to the left-eye image data and the right-eye image (second image) corresponding to the right-eye image data. The view angle correction data includes a magnification γ (first magnification) of the view angle of the right-eye image relative to the view angle of the left-eye image. The memory 24 stores position data indicating a position of a reference area (extraction reference area) which indicates a size of a reference of the extraction area from the respective images in the light source integrated-type processor 3. The position data includes first position data indicating a position of a reference area in the left-eye image and second position data indicating a position of a reference area in the right-eye image. Incidentally, the memory 24 may record various parameters for image processing with respect to image data captured by the left-eye image sensor 22A and the right-eye image sensor 22B, such as a parameter for white balance (WB) adjustment. Various types of information recorded by the memory 24 are output to a correction data acquisition unit 311 of the light source integrated-type processor 3 via an electrical cable (not illustrated) through a communication process with the light source integrated-type processor 3 when the endoscope 2 is mounted to the light source integrated-type processor 3.

The illumination lens 25 is positioned at a distal end of the light guide cable extending from the connector. When the endoscope 2 is mounted to the light source integrated-type processor 3, light generated from the light source unit 6, which will be described later, of the light source integrated-type processor 3, is illuminated from the illumination lens 25 at the distal end of the endoscope 2 to the subject via the light guide cable.

The endoscope 2 is mounted to the light source integrated-type processor 3 in a detachable manner, and the light source integrated-type processor 3 performs the predetermined image processing on the left-eye image data and the right-eye image data transmitted from the mounted endoscope 2 to generate the stereoscopic image. The light source integrated-type processor 3 outputs the generated stereoscopic image to the display device 4 to be displayed.

The light source integrated-type processor 3 includes a control unit 31, a storage unit 32 (second storage unit), a right and left image converter 33, a first image processing unit 34, the correction unit 35, a second image processing unit 36, an OSD (on-screen display) generation unit 37, a composing unit 38, a display controller 39, and a light source unit 6.

The control unit 31 is implemented using a CPU and the like. The control unit 31 controls processing operations of the respective parts of the light source integrated-type processor 3 by performing transfer of the instruction information and data with respect to the respective configuration of the light source integrated-type processor 3. When the endoscope 2 is mounted to the light source integrated-type processor 3, the control unit 31 is connected to each of the left-eye image sensor 22A, the right-eye image sensor 22B, the left-eye signal processing unit 23A, and the right-eye signal processing unit 23B of the endoscope 2 via each cable, and also controls these parts. The control unit 31 includes a correction data acquisition unit 311 and a parameter generation unit 312.

The correction data acquisition unit 311 acquires the view angle correction data from the memory 24 of the endoscope 2 which is actually mounted to the light source integrated-type processor 3. The correction data acquisition unit 311 acquires the instruction data to instruct the correction unit 35 to cause the view angle and the size of the right-eye image to match the view angle and the size of the left-eye image, the magnification γ of the view angle of the right-eye image relative to the view angle of the left-eye image of the endoscope 2, the first position data indicating the position of the reference area in the left-eye image, and a second position data indicating a position of the reference area in the right-eye image, from the memory 24. The correction data acquisition unit 311 acquires the identification information indicating the type of the endoscope 2 from the memory 24 of the endoscope 2, and acquires a magnification α (second magnification) according to the type of the endoscope 2 indicated by the acquired identification information and a standard of the display device 4 from the storage unit 32 to be described later. The magnification α is a scale factor with respect to an image as a scaling process target, which is set in advance according to the type of the endoscope 2 and the standard of the display device 4. The magnification α is used in a process of enlarging or reducing an input image in order to generation an image having a size that matches a size of the image displayed by the display device 4. In the embodiment, the following description is given assuming that the magnification α is a scale factor with respect to the left-eye image, and the scale factor with respect to the right-eye image is also α.

The parameter generation unit 312 generates, based on the magnification γ acquired by the correction data acquisition unit 311, a first scale factor, which is used with respect to a first extraction area extracted from the left-eye image, in the scaling process executed by a scaling unit 352 to be described later. The parameter generation unit 312 generates, based on the magnification γ and the magnification α acquired by the correction data acquisition unit 311, a second scale factor, which is used with respect to a second extraction area extracted from the right-eye image, in the scaling process executed by the scaling unit 352 to be described later.

The storage unit 32 is implemented using a volatile memory or a non-volatile memory, and stores various programs for operation of the light source integrated-type processor 3. The storage unit 32 temporarily records information in the middle of being processed by the light source integrated-type processor 3. The storage unit 32 stores information indicating the reference area which indicates a reference size of the extraction area from each image. The storage unit 32 stores scale factor data 321 in which the magnification α is associated with each of the type of the endoscope 2 and the standard of the display device 4. The storage unit 32 stores the various types of information, such as the left-eye image data, the right-eye image data, and the identification information, output from the endoscope 2. The storage unit 32 may be configured using a memory card and the like which is mounted from the outside of the light source integrated-type processor 3.

The right and left image converter 33, based on the left-eye image data input from the left-eye signal processing unit 23A and the right-eye image data input from the right-eye signal processing unit 23B, arranges the left-eye image corresponding to the left-eye image data and the right-eye image corresponding to the right-eye image data side by side to be converted single image data using, for example, a Side-by-Side system. The right and left image converter 33 converts the left-eye image data input from the left-eye signal processing unit 23A and the right-eye image data input from the right-eye signal processing unit 23B into a format which is suitable for the image processing in the first image processing unit 34 in the subsequent stage.

The first image processing unit 34 performs an optical black (OB) subtraction process, a demosaicing process, a white balance (WB) adjustment process, and the like on image data input from the right and left image converter 33.

The correction unit 35 corrects at least any one of the left-eye image data and the right-eye image data based on the view angle correction data acquired by the correction data acquisition unit 311 such that the view angle of the left-eye image corresponding to the left-eye image data and the view angle of the right-eye image corresponding to the right-eye image data match each other, and the size of the left-eye image and the size of the right-eye image match each other. In the embodiment, the correction unit 35 is instructed to cause the view angle and the size of the right-eye image to match the view angle and the size of the left-eye image using the instruction data. Incidentally, the following description is given assuming that sizes of two images match each other when sizes in the horizontal direction match each other and sizes in the vertical direction match each other between the two images. The correction unit 35 includes a processing area setting unit 351 and the scaling unit 352.

The processing area setting unit 351 sets the position of the reference area in the left-eye image based on the first position data acquired by the correction data acquisition unit 311. The processing area setting unit 351 sets the position of the reference area in the right-eye image based on the second position data acquired by the correction data acquisition unit 311. The processing area setting unit 351 sets a size of the first extraction area extracted from the left-eye image based on the set reference area of the left-eye image, and sets a size of the second extraction area extracted from the right-eye image based on the set reference area of the right-eye image and the magnification γ acquired by the correction data acquisition unit 311.

The scaling unit 352 extracts the first extraction area from the left-eye image and outputs an image obtained by enlarging or reducing the extracted first extraction area using the first scale factor, as a corrected left-eye image. The scaling unit 352 extracts the second extraction area from the right-eye image and outputs an image obtained by enlarging or reducing the extracted second extraction area using the second scale factor, as a corrected right-eye image. The first scale factor and the second scale factor are generated by the parameter generation unit 312.

The second image processing unit 36 performs image processing such as a structure enhancement process (edge enhancement process) on the corrected right-eye image and the corrected left-eye image, which are output from the correction unit 35, to generate image data for the image data for stereoscopic display.

The OSD generation unit 37 generates image data, such as a letter and a menu, to be superimposed on the image data for stereoscopic display.

The composing unit 38 generates image data for display obtained by composing an image generated by the OSD generation unit 37 with the image data for stereoscopic display output from the second image processing unit 36.

The display controller 39 converts the image data for display generated by the composing unit 38 into image data in a format that can be displayed and output by the display device 4, and causes the display device 4 to display the converted image data. The display controller 39 includes a converter (DAC) or an encoder to convert a digital signal into an analog signal, and converts the image data input from the composing unit 38, for example, from the digital signal into the analog signal, changes the converted image data of the analog signal into a format of a high vision system and the like, and outputs the changed data to the display device 4.

The light source unit 6 includes a light source driver and a light source, and supplies illumination light to the endoscope 2 under control of the control unit 31. The light source of the light source unit 6 includes a white LED that generates white light, for example. In addition, the light source of the light source unit 6 may use a plurality of LED's (for example, a red LED, a green LED, and a blue LED) generating light having different wavelength bands and obtain illumination light having a desired color tone by combining the light generated by the respective LED's. In addition, the light source unit 6 may adopt a sequential lighting configuration in which beams of light having different color components are emitted in a time-series manner. In addition, the light source unit 6 may use a laser light source. In addition, the light source unit 6 may be configured to include a light source control parts to control the light source, such as a xenon lamp and a halogen lamp, an optical filter, a diaphragm, and each member of the light source unit 6.

The display device 4 is configured using a display and the like which employs a liquid crystal or organic EL (Electro Luminescence). The display device 4 displays various types of information including the image for display output from the light source integrated-type processor 3.

The input device 5 is implemented using an operation device such as a mouse, a keyboard, and a touch panel, receives input of various types of instruction data, and inputs the received the various types of instruction data to the control unit 31 of the light source integrated-type processor 3. The input device 5 receives input of data such as patient data (for example, an ID, a date of birth, a name, and the like) relating to a patient serving as a subject and content of inspection.

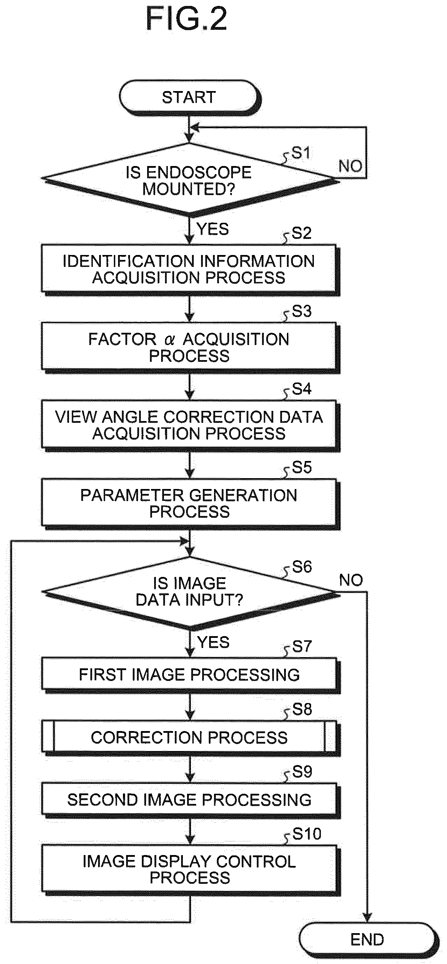

FIG. 2 is a flowchart illustrating a processing procedure of a process executed with respect to the image data input from the endoscope 2 by the light source integrated-type processor 3.

As illustrated in FIG. 2, the control unit 31 determines whether the endoscope 2 is mounted to the light source integrated-type processor 3 in the light source integrated-type processor 3 (Step S1). When determining that the endoscope 2 is not mounted to the light source integrated-type processor 3 (Step S1: No), the control unit 31 repeats the determination process in Step S1 until determining that the endoscope 2 is mounted to the light source integrated-type processor 3.

When the control unit 31 determines that the endoscope 2 is mounted to the light source integrated-type processor 3 (Step S1: Yes), the correction data acquisition unit 311 performs the communication process betwenn the correction data acquisition unit 311 and the memory 24 of the endoscope 2 and performs an identification information acquisition process of acquiring the identification information of the endoscope 2 from the memory 24 (Step S2).

The correction data acquisition unit 311 performs a magnification α acquisition process of reading and acquiring the magnification α according to the type of the endoscope 2 indicated by the identification information acquired in Step S2 and the standard of the display device 4 connected to the light source integrated-type processor 3, from the scale factor data 321 of the storage unit 32 (Step S3) .

The correction data acquisition unit 311 performs a view angle correction data acquisition process of acquiring the view angle correction data, which includes the instruction data, the magnification γ, the first position data, and the second position data, from the memory 24 (Step S4). Step S2 and Step S4 may be processed in parallel. The magnification α and the view angle correction data, acquired by correction data acquisition unit 311, are output to the correction unit 35.

The parameter generation unit 312 performs a parameter generation process of generating the first scale factor based on the magnification γ acquired by the correction data acquisition unit 311, and generating the second scale factor based on the magnification γ and the magnification α acquired by the correction data acquisition unit 311 (Step S5). The first scale factor and the second scale factor generated by the parameter generation unit 312 are output to the scaling unit 352 of the correction unit 35.

The control unit 31 determines whether the image data is input from the endoscope 2 (Step S6). When determining that the image data is not input from the endoscope 2 (Step S6: No), the control unit 31 ends the process.

When the control unit 31 determines that the image data is input from the endoscope 2 (Step S6: Yes), the right and left image converter 33 converts the left-eye image data and the right-eye image data as the input image data into the single image data, and then, the first image processing unit 34 performs first image processing such as the OB subtraction process, the demosaicing process, and the WB adjustment process (Step S7).

The correction unit 35 performs the correction process of causing the view angle of the left-eye image corresponding to the left-eye image data and the view angle of the right-eye image corresponding to the right-eye image data to match each other and causing the size of the left-eye image and the size of the right-eye image to match each other with respect to the image on which the first image processing has been performed(Step S8). The left-eye image and right-eye image corrected by the correction unit 35 are images whose view angles and image sizes match each other.

The second image processing unit 36 and the composing unit 38 perform second image processing of performing the image processing such as the edge enhancement process on the left-eye image and right-eye image corrected by the correction unit 35 to generate the image data for stereoscopic display (Step S9). The display controller 39 performs an image display control process of converting the image data for stereoscopic display generated in the second image processing into image data in the format that can be displayed and output by the display device 4, and causing the display device 4 to display the converted image data (Step S10). Accordingly, the stereoscopic image, obtained based on the left-eye image and the right-eye image whose view angles and image sizes match each other, is displayed on the display device 4. Thereafter, the process returns to Step S6 and is continued.

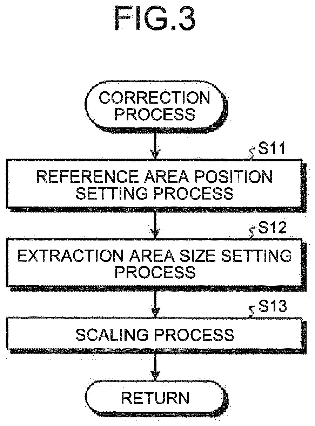

FIG. 3 is a flowchart illustrating a process procedure of the correction process illustrated in FIG. 2. As illustrated in FIG. 3, the processing area setting unit 351 performs a reference area position setting process of setting the position of the reference area in the left-eye image based on the first position data acquired by the correction data acquisition unit 311 and setting the position of the reference area in the right-eye image based on the second position data in the correction process (Step S11).

The processing area setting unit 351 performs an extraction area size setting process of setting the size of the first extraction area to be extracted from the left-eye image and setting the size of the second extraction area to be extracted from the right-eye image (Step S12).

The scaling unit 352 performs the scaling process of extracting the first extraction area from the left-eye image and outputting the image obtained by enlarging or reducing the extracted first extraction area using the first scale factor as the corrected left-eye image, and further extracting the second extraction area from the right-eye image and outputting the image obtained by enlarging and reducing the extracted second extraction area using the second scale factor as the corrected right-eye image (Step S13).

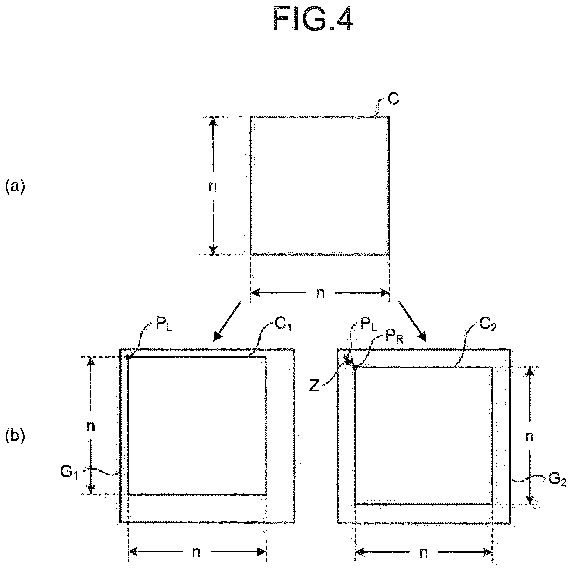

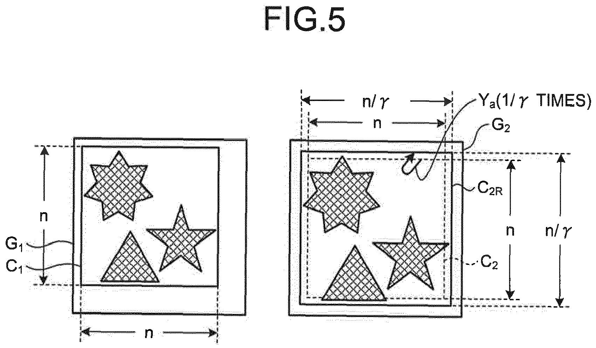

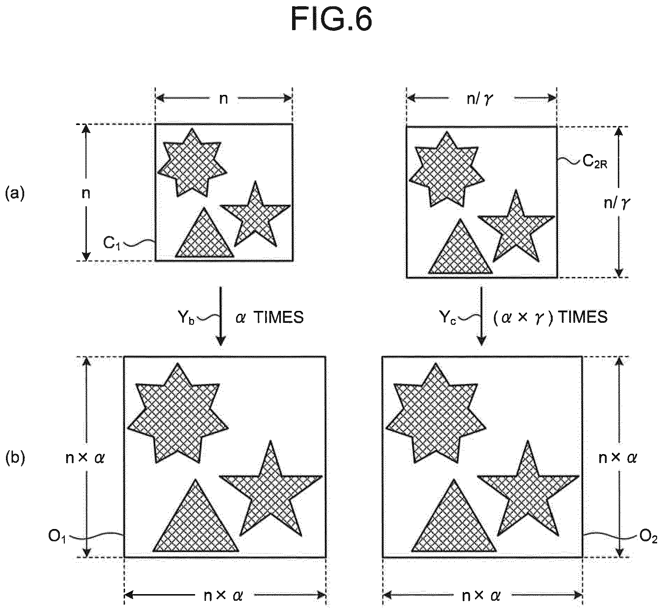

The respective processes illustrated in FIG. 3 will be described with reference to FIGS. 4 to 6. FIG. 4 is a view for describing the reference area position setting process illustrated in FIG. 3. FIG. 5 is a view for describing the extraction area size setting process illustrated in FIG. 3. FIG. 6 is a view for describing the scaling process illustrated in FIG. 3.

A description will be given regarding an example assuming a case where a square-shaped area C in which both sizes in the horizontal direction and the vertical direction are n is set as the reference area size, the case where the first position data indicates a position P_{L} of an upper left vertex of the reference area in the left-eye image and the second position data indicates a position P_{R} of an upper left vertex of the reference area in the right-eye image as illustrated in (a) of FIG. 4, for example. The processing area setting unit 351 sets an area C₁ by arranging the area C such that the upper left vertex of the area C is positioned at the position P_{L} indicated by the first position data, as a reference area in a left-eye image G₁ in the reference area position setting process (Step S11) as illustrated in (b) of FIG. 4. The processing area setting unit 351 sets an area C₂ by arranging the area C such that the upper left vertex of the area C is positioned at the position P_{R} indicated by the second position data, as a reference area in a right-eye image G₂ as illustrated in (b) of FIG. 4. Incidentally, the second position data may be a deviation amount Z with respect to the upper left vertex P_{L} of the reference area in the left-eye image. In this case, the processing area setting unit 351 may set the reference area C₂ by arranging the area C such that the upper left vertex of the area C is positioned at the position deviated from the position P_{L} by the amount Z.

In the embodiment, it is instructed to cause the view angle and the size of the right-eye image to match the view angle and the size of the left-eye image. The processing area setting unit 351 sets the reference area C₁ set in Step S11 directly as the first extraction area with respect to the left-eye image G₁ in the extraction area size setting process (Step S12) as illustrated in FIG. 5. The processing area setting unit 351 sets an area C_{2R}, obtained by enlarging or reducing the reference area C₂ of the right-eye image G₂ set in Step S11 by (1/γ) times in the horizontal direction and the vertical direction, as indicated by an arrow Yₐ, as the second extraction area with respect to the right-eye image G₂ in order to cause the view angle of the right-eye image to match the view angle of the left-eye image. Accordingly, the square-shaped second extraction area C_{2R} with the sizes in both the horizontal direction and the vertical direction of (n/γ) is set. Incidentally, the processing area setting unit 351 enlarges or reduces the reference area C₂ both in the horizontal direction and the vertical direction by (1/γ) times in the state of fixing a position of the center of the reference area C₂.

In the scaling process (Step S13), the scaling unit 352 extracts the first extraction area C₁ from the left-eye image G₁ (see FIG. 5) and extracts the second extraction area C_{2R} from the right-eye image G₂ (see FIG. 5) as illustrated in (a) of FIG. 6. When the square-shaped area C is set as the reference area size as illustrated in (a) of FIG. 4, the parameter generation unit 312 sets the magnification α for the first scale factor with respect to the left-eye image that is not the correction target in Step S5. The parameter generation unit 312 sets a magnification (α×γ), obtained by multiplying the magnification α with the magnification γ, as the second scale factor with respect to the right-eye image serving as the correction target such that the corrected size of the left-eye image, enlarged or reduced using the magnification α, and the corrected size of the right-eye image match each other. Accordingly, the scaling unit 352 generates an enlarged or reduced image O₁ by enlarging or reducing each of the size in the horizontal direction and the size in the vertical direction of the first extraction area C₁, extracted from the left-eye image G₁, by α times as indicated by an arrow Y_{b} (see (b) of FIG. 6). The scaling unit 352 generates an enlarged or reduced image O₂ by enlarging or reducing each of the size in the horizontal direction and the size in the vertical direction of the extracted second extraction area C_{2R}, by (α × γ) times as indicated by an arrow Y_{c} (see (b) of FIG. 6). As a result, the left-eye image O₁ and the right-eye image O₂ have both the sizes of the horizontal direction and the vertical direction of (n × α), and the image sizes thereof match each other.

In this manner, the present embodiment causes the memory 24 of each of the endoscopes 2 to store the view angle correction data for correction of the difference between the view angle of the left-eye optical system 21A and the view angle of the right-eye optical system 21B of the endoscope 2. When the endoscope 2 is mounted to the light source integrated-type processor 3, the light source integrated-type processor 3 reads the view angle correction data from the memory 24 of the endoscope 2, and executes the correction process based on the read view angle correction data such that the view angle of the left-eye image corresponding to the left-eye image data and the view angle of the right-eye image corresponding to the right-eye image data match each other, and the size of the left-eye image and the size of the right-eye image match each other. In other words, the light source integrated-type processor 3 reads the view angle correction data corresponding to the endoscope 2, which has been actually mounted, from the memory 24 of the endoscope 2 and executes the correction process based on the read view angle correction data even when the variation in size between the images caused by the individual differences of the two optical systems for stereoscopic observation differs depending on each of the endoscopes 2 in the embodiment. Accordingly, the light source integrated-type processor 3 can suitably execute the image correction in response to the mounted endoscope 2 even if an arbitrary kind of the endoscope 2 is mounted to the light source integrated-type processor 3 according to the embodiment.

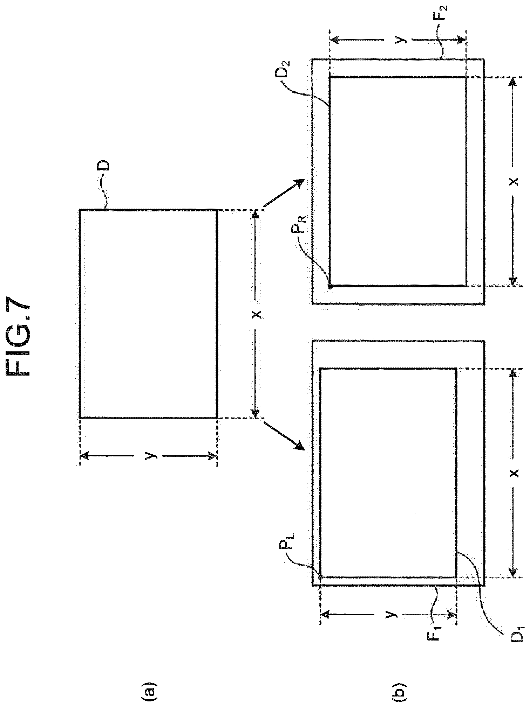

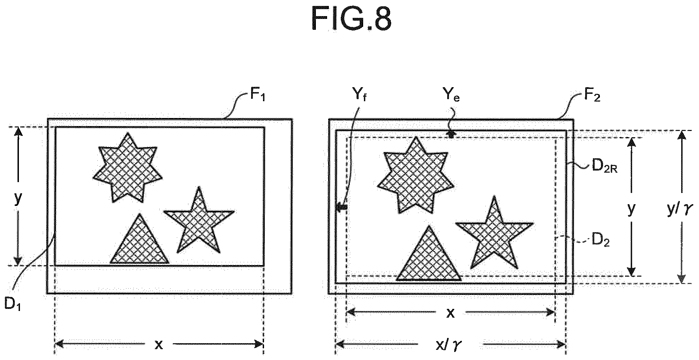

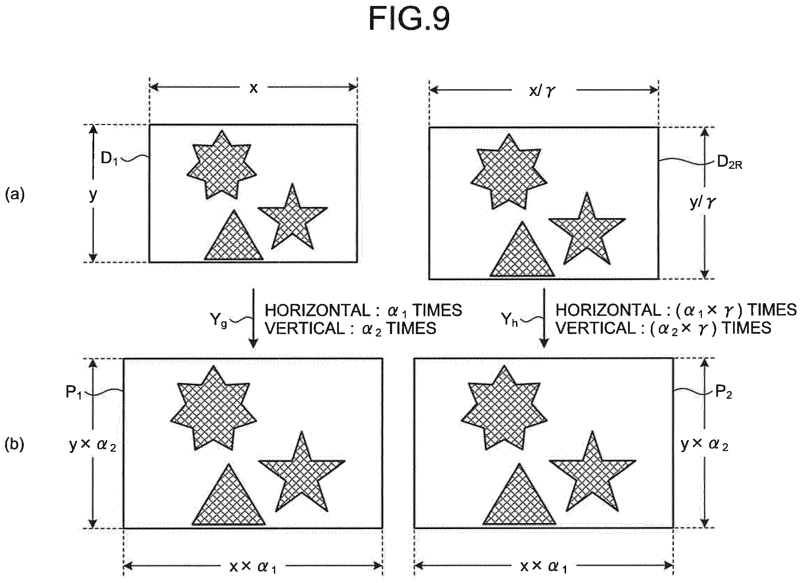

Incidentally, when a rectangular-shaped area having different sizes in the horizontal direction and the vertical direction is set as a reference area size, there is a case where a magnification α₁ is set for the horizontal direction and a magnification α₂ is set for the vertical direction as the first magnification. A correction process in this case will be described. FIG. 7 is a view for describing the reference area position setting process illustrated in FIG. 3. FIG. 8 is a view for describing the extraction area size setting process illustrated in FIG. 3. FIG. 9 is a view for describing the scaling process illustrated in FIG. 3.

As illustrated in (a) of FIG. 7, for example, a rectangular-shaped area D whose size in the horizontal direction is x and size in the vertical direction is y is set as the reference area size. The processing area setting unit 351 sets an area D₁ by arranging the area D such that the upper left vertex of the area D is positioned at the position P_{L} as a reference area in a left-eye image F₁, and sets an area D₂ by arranging the area D such that the upper left vertex of the area D is positioned at the position P_{R} as a reference area in a right-eye image F₂ in the reference area position setting process (Step S11) as illustrated in (b) of FIG. 7.

The processing area setting unit 351 sets the reference area D₁ set in Step S11 directly as the first extraction area with respect to the left-eye image F₁ in the extraction area size setting process (Step S12) as illustrated in FIG. 8. The processing area setting unit 351 sets an area D_{2R}, obtained by enlarging or reducing the reference area D₂ of the right-eye image F₂ set in Step S11 by (1/γ) times in the horizontal direction and the vertical direction, as indicated by an arrows Yₑ and Y_{f}, as the second extraction area with respect to the right-eye image F₂ in order to cause the view angle of the right-eye image to match the view angle of the left-eye image. Accordingly, the rectangular-shaped second extraction area D_{2R} is set such that the size in the horizontal direction is (x/γ) and the size in the vertical direction is (y/γ), with respect to the right-eye image F₂.

In the scaling process (Step S13), the scaling unit 352 extracts the first extraction area D₁ from the left-eye image F₁ (see FIG. 8), and extracts the second extraction area D_{2R} from the right-eye image F₂ (see FIG. 8) as illustrated in (a) of FIG. 9. Here, in Step S5, the parameter generation unit 312 sets the magnification in the horizontal direction as α₁ and the magnification in the vertical direction as α₂ as the first scale factor with respect to the left-eye image that is not the correction target. On the other hand, in Step S5, the parameter generation unit 312 sets a magnification obtained by multiplying the respective magnifications α₁ and α₂ with the magnification γ as the second scale factor with respect to the right-eye image serving as the correction target. To be specific, the parameter generation unit 312 sets the magnification in the horizontal direction as (α₁ × γ), and the magnification in the vertical direction as (α₂ × γ) as the second scale factor. Accordingly, the scaling unit 352 generates an image P₁ obtained by enlarging or reducing the first extraction area D₁ in the horizontal direction by α₁ times and in vertical direction by α₂ times as indicated by an arrow Y_{g} (see (b) of FIG. 9). The scaling unit 352 generates an image P₂ obtained by enlarging or reducing the second extraction area D_{2R} in the horizontal direction by (α₁ × γ) times and in vertical direction by (α₂ × γ) times as indicated by an arrow Yₕ (see (b) of FIG. 9). As a result, both the corrected left-eye image P₁ and the corrected right-eye image P₂ have the size in the horizontal direction of (x×α₁) and the size in the vertical direction of (y×α₂), and thus, the image sizes thereof match each other.

In addition, the description has been given in the embodiment regarding the example in which the left-eye image data and the right-eye image data are acquired by providing the two image sensors, but it may be configured such that a light receiving surface of a CMOS image sensor is divided into two areas of a left-eye area and a right-eye area and left-eye image data and right-eye image data are acquired using the single CMOS image sensor.

In addition, the description has been given in the embodiment regarding the example where the sizes of the right and left images are set to match each other through the scaling process with respect to the image data performed by the scaling unit 352, but the control unit 31 may perform the correction process of causing the view angles and the sizes of the right and left images to match each other by controlling at least one of optical zoom functions of the left-eye optical system 21A and the right-eye optical system 21B based on the parameter generated by the parameter generation unit 312.

In addition, the description has been given in the embodiment by exemplifying the light source integrated-type processor 3 integrated with the light source unit 6, but the present invention may be applied in the same manner even in a case where a processor and a light source device are separately provided.

In addition, a signal that is transmitted and received between the endoscope 2 and the light source integrated-type processor 3 in the embodiment is not necessarily the electrical signal but may be an optical signal obtained by conversion of the electrical signal. In this case, the transmission of the optical signal is performed between the endoscope 2 and the light source integrated-type processor 3 using a transmission path for the optical signal such as an optical fiber. The transmission and reception of the signal may be performed between the endoscope 2 and the light source integrated-type processor 3 using wireless communication without being limited to wired communication.

In addition, the description has been given in the embodiment regarding the endoscopic system to which the endoscope including the flexible insertion portion is applied, but an endoscopic system to which an endoscope including a rigid insertion portion is applied may be implemented. In addition, the endoscope that functions as the imaging device may have a light source and a control function to control the image sensor and the light source. In this case, the light source may be a semiconductor light source or the like which is provided at a distal end of the insertion portion of the endoscope. In addition, it may be configured such that an image sensor is provided at a proximal end of an insertion portion to capture an optical image transmitted via an optical fiber from a distal end to the proximal end of the insertion portion, for example, without being limited to the configuration where the image sensor is provided at the distal end of the insertion portion of the endoscope. In addition, it may be configured to connect an eyepiece camera head of an optical endoscope such as a fiber scope and a telescope without being limited to the endoscope provided with the image sensor at the distal end of the insertion portion.

In addition, the description has been given by exemplifying, the endoscopic system 1 as the embodiment, but the invention is not limited to the endoscopic system, and can be also applied to an imaging system in which an imaging unit, which includes the left-eye optical system 21A, the right-eye optical system 21B, the imaging unit 20, the memory 24, and the illumination lens 25, is mounted to a processing device, which includes at least the control unit 31, the storage unit 32, the right and left image converter 33, the first image processing unit 34, the correction unit 35, and the second image processing unit 36, in a detachable manner.

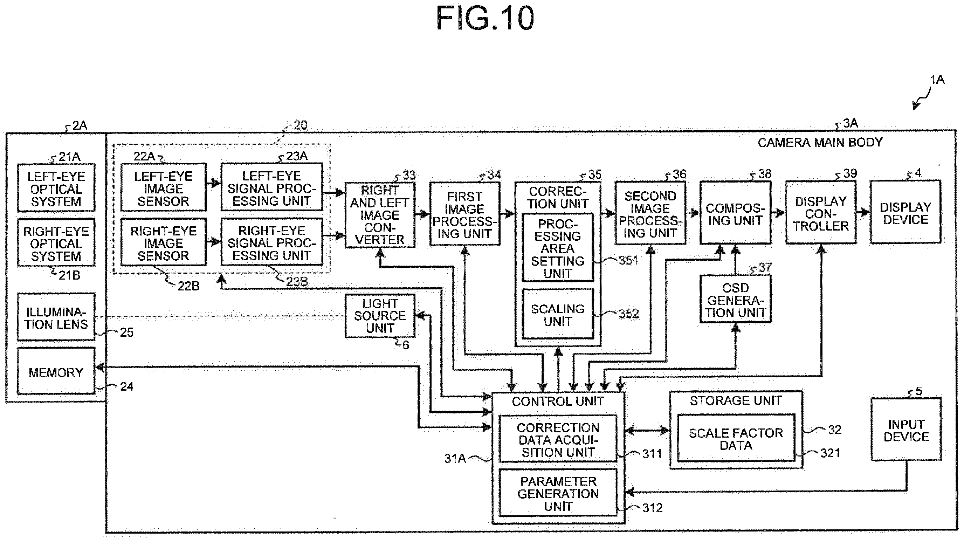

FIG. 10 is a schematic view illustrating another schematic configuration of an imaging system according to an embodiment of the invention. The imaging system according to the present embodiment may have a configuration where the imaging unit 20 is provided in a camera main body 3A which includes the display device 4 and the input device 5 as illustrated in an imaging system 1A in FIG. 10. In this case, an optical unit 2A including the left-eye optical system 21A, the right-eye optical system 21B, the memory 24, and the illumination lens 25 is mounted to the camera main body 3A in a detachable manner, and the correction data acquisition unit 311 of a control unit 31A reads view angle correction data corresponding to the optical unit 2A from the memory 24 of the optical unit 2A in the camera main body 3A.

In addition, the light source integrated-type processor, the camera main body, and the execution programs relating to the respective processed executed by the other configuration units according to the present embodiment may be provided by being recorded in a computer-readable recording medium, such as a CD-ROM, a flexible disc, a CD-R, and a DVD, in an installable or executable file format or may be stored in a computer connected to a network, such as the Internet to be provided via download through the network. In addition, it may be configured to be provided or distributed through the network such as the Internet. Reference Signs List

- 1: ENDOSCOPIC SYSTEM
- 1A: IMAGING SYSTEM
- 2: ENDOSCOPE
- 2A: OPTICAL UNIT
- 3: LIGHT SOURCE INTEGRATED-TYPE PROCESSOR
- 3A: CAMERA MAIN BODY
- 4: DISPLAY DEVICE
- 5: INPUT DEVICE
- 6: LIGHT SOURCE UNIT
- 20: IMAGING UNIT
- 21A: LEFT-EYE OPTICAL SYSTEM (FIRST OPTICAL SYSTEM)
- 21B: RIGHT-EYE OPTICAL SYSTEM (SECOND OPTICAL SYSTEM)
- 22A: LEFT-EYE IMAGE SENSOR (FIRST IMAGING UNIT)
- 22B: RIGHT-EYE IMAGE SENSOR (SECOND IMAGING UNIT)
- 23A: LEFT-EYE SIGNAL PROCESSING UNIT
- 23B: RIGHT-EYE SIGNAL PROCESSING UNIT
- 24: MEMORY
- 25: ILLUMINATION LENS
- 31, 31A: CONTROL UNIT
- 32: STORAGE UNIT
- 33: RIGHT AND LEFT IMAGE CONVERTER
- 34: FIRST IMAGE PROCESSING UNIT
- 35: CORRECTION UNIT
- 36: SECOND IMAGE PROCESSING UNIT
- 37: OSD GENERATION UNIT
- 38: COMPOSING UNIT
- 39: DISPLAY CONTROLLER
- 311: CORRECTION DATA ACQUISITION UNIT
- 312: PARAMETER GENERATION UNIT
- 321: SCALE FACTOR DATA
- 351: PROCESSING AREA SETTING UNIT
- 352: SCALING UNIT

## Claims

1. An imaging system comprising:
an optical unit;
a processing device having a configuration to which the optical unit is mounted in a detachable manner; and
an imaging device which is arranged in either the optical unit or the processing device, wherein
the optical unit includes:
a first optical system configured to image light incident from a subject;
a second optical system configured to image the light incident from the subject with a parallax from the first optical system; and
a first storage unit configured to store view angle correction data for correction of a difference between a view angle of the first optical system and a view angle of the second optical system,
the imaging device includes:
a first imaging unit configured to capture an optical image imaged by the first optical system to generate first image data; and
a second imaging unit configured to capture an optical image imaged by the second optical system to generate second image data, and
the processing device includes:
a data acquisition unit configured to acquire the view angle correction data from the first storage unit; and
a correction unit configured to correct at least one of the first image data and the second image data based on the view angle correction data acquired by the data acquisition unit such that a view angle of a first image corresponding to the first image data and a view angle of a second image corresponding to the second image data match each other, and a size of the first image and a size of the second image match each other.

2. The imaging system according to claim 1, wherein
the view angle correction data includes at least instruction data, which instructs the correction unit to cause the view angle and the size of the second image to match the view angle and the size of the first image, and a first magnification of the view angle of the second image relative to the view angle of the first image.

3. The imaging system according to claim 2, wherein
the processing device further includes:
a display unit configured to display and output an image processed by the processing device; and
a parameter generation unit configured to generate a first scale factor to be used in the correction unit based on a second magnification, which is a scale factor set in advance according to a type of the optical unit and a standard of the display unit, and a second scale factor to be used in the correction unit based on the first magnification acquired by the data acquisition unit and the second magnification, and
the correction unit includes:
an area setting unit configured to set a size of a first extraction area to be extracted from the first image based on an extraction reference area from each image, and set a size of a second extraction area to be extracted from the second image based on the extraction reference area and the first magnification acquired by the data acquisition unit; and
a scaling unit configured to extract the first extraction area from the first image and output, as a corrected first image, an image obtained by enlarging or reducing the extracted first extraction area using the first scale factor, and further, extract the second extraction area from the second image and output, as a corrected second image, an image obtained by enlarging or reducing the extracted second extraction area using the second scale factor.

4. The imaging system according to claim 3, wherein
the processing device further includes a second storage unit configured to store the second magnification to be associated with each of the type of the optical unit and the standard of the display unit,
the first storage unit is configured to store identification information indicating the type of the optical unit,
the data acquisition unit is configured to acquire the identification information of the optical unit from the first storage unit, and acquire the second magnification according to the type of the optical unit and the standard of the display unit indicated by the acquired identification information, from the second storage unit, and
the parameter generation unit is configured to generate the first scale factor and the second scale factor based on the first magnification and the second magnification acquired by the data acquisition unit.

5. The imaging system according to claim 3 or 4, wherein
the view angle correction data includes first position data indicating a position of the extraction reference area in the first image and second position data indicating a position of the extraction reference area in the second image, and
the area setting unit is configured to set the position of the extraction reference area in the first image based on the first position data acquired by the data acquisition unit and set the position of the extraction reference area in the second image based on the second position data acquired by the data acquisition unit.

6. The imaging system according to claim 1 or 2, wherein
each of the first optical system and the second optical system has an optical zoom function to change the view angle, and
the correction unit is configured to control the optical zoom function of at least any one of the first optical system and the second optical system based on the view angle correction data acquired by the data acquisition unit so as to cause the view angle of the first image corresponding to the first image data and the view angle of the second image corresponding to the second image data to match each other and the size of the first image and the size of the second image to match each other.

7. The imaging system according to any one of claims 1 to 6, comprising:
an endoscopic device including the optical unit and the imaging device; and
the processing device configured to process image data captured by the imaging device.

8. A processing device to which an optical unit is mounted in a detachable manner and configured to process image data output from an imaging device, the optical unit including a first optical system configured to image light incident from a subject, a second optical system configured to image the light incident from the subject with a parallax from the first optical system, and a first storage unit configured to store view angle correction data for correction of a difference between a view angle of the first optical system and a view angle of the second optical system, the imaging device being arranged in either the optical unit or the processing device and including a first imaging unit configured to capture an optical image imaged by the first optical system to generate first image data and a second imaging unit configured to capture an optical image imaged by the second optical system to generate second image data, the processing device comprising:
a data acquisition unit configured to acquire the view angle correction data from the first storage unit of the optical unit mounted to the processing device; and
a correction unit configured to correct at least one of the first image data and the second image data based on the view angle correction data acquired by the data acquisition unit such that a view angle of a first image corresponding to the first image data and a view angle of a second image corresponding to the second image data match each other, and a size of the first image and a size of the second image match each other.

9. A processing method that is executed by a processing device to which an optical unit including a first optical system that images light incident from a subject and a second optical system that images the light incident from the subject with a parallax from the first optical system is mounted in a detachable manner, the processing device being configured to process image data output from a first imaging unit configured to capture an optical image imaged by the first optical system to generate first image data and a second imaging unit configured to capture an optical image imaged by the second optical system to generate second image data, the processing method comprising:
a data acquisition process of acquiring view angle correction data for correction of a difference between a view angle of the first optical system and a view of the second optical system, from a first storage unit included in the optical unit mounted to the processing device; and
a correction process of correcting at least one of the first image data and the second image data based on the view angle correction data acquired in the data acquisition process such that a view angle of a first image corresponding to the first image data and a view angle of a second image corresponding to the second image data match each other, and a size of the first image and a size of the second image match each other.

10. A processing program that causes a processing device to which an optical unit including a first optical system that images light incident from a subject and a second optical system that images the light incident from the subject with a parallax from the first optical system is mounted in a detachable manner, the processing device being configured to process image data output from a first imaging unit configured to capture an optical image imaged by the first optical system to generate first image data and a second imaging unit configured to capture an optical image imaged by the second optical system to generate second image data, to execute:
a data acquisition procedure of acquiring view angle correction data for correction of a difference between a view angle of the first optical system and a view angle of the second optical system, from a first storage unit included in the optical unit mounted to the processing device; and
a correction procedure of correcting at least one of the first image data and the second image data based on the view angle correction data acquired in the data acquisition procedure such that a view angle of a first image corresponding to the first image data and a view angle of a second image corresponding to the second image data match each other, and a size of the first image and a size of the second image match each other.
